# EUROPEAN PATENT SPECIFICATION

(11) **EP 4 420 754 B1**
(45) Date of publication and mention of the grant of the patent: **25.02.2026**
(21) Application number: 23889852.2
(22) Date of filing: 25.05.2023
(51) Int. Cl.: B01D 9/02, B01D 9/00, B01D 61/02, B01D 61/58, B01F 21/00, B01F 33/82, B01J 47/02, A23L 27/30, B01F 101/06, C07C 29/78, C07H 1/00, C13B 30/02, C13B 30/06, C13B 30/12, C30B 7/02, C30B 7/10, C30B 29/54

(54) **PREPARATION SYSTEM FOR COMPOUND CRYSTALS OF ERYTHRITOL AND HIGH-INTENSITY SWEETENERS AND METHOD THEREFOR**
SYSTEM ZUR HERSTELLUNG VON ERYTHRITOLVERBINDUNGSKRISTALLEN UND SÜSSMITTELN MIT HOHER SÜSSKRAFT UND VERFAHREN DAFÜR
SYSTÈME DE PRÉPARATION DE CRISTAUX COMPOSÉS D'ÉRYTHRITOL ET D'ÉDULCORANTS À HAUTE INTENSITÉ ET PROCÉDÉ ASSOCIÉ

(30) Priority: 25.12.2022 CN 202211669958
(43) Date of publication of application: 28.08.2024
(73) Proprietor: Zhejiang Huakang Pharmaceutical Co., Ltd., Quzhou, Zhejiang 324302 (CN)
(72) Inventor: QIN, Shufang, Quzhou, Zhejiang 324302 (CN); LI, Mian, Santa Clara, CA (US); ZHANG, Wenyao, Quzhou, Zhejiang 324302 (CN); YANG, Wulong, Quzhou, Zhejiang 324302 (CN); WU, Qiang, Quzhou, Zhejiang 324302 (CN); ZHEN, Ni, Quzhou, Zhejiang 324302 (CN); XU, Weidong, Quzhou, Zhejiang 324302 (CN)
(74) Representative: Sun, Yiming
(86) International application number: PCT/CN2023/096372
(87) International publication number: WO 2024/139030

(56) References cited:
- EP-A2- 0 525 659
- CN-A- 102 742 761
- CN-A- 113 248 551
- CN-A- 113 248 551
- CN-A- 115 804 966
- US-A1- 2013 344 216

## Description

### TECHNICAL FIELD

The present invention relates to a system and a method for preparing a compound crystal of erythritol and a high-intensity sweetener. The invention is set out in the appended claims.

### BACKGROUND

Erythritol is a natural sugar alcohol sweetener with many advantages, such as pure sweetness, a low calorific value, non-cariogenic property, refreshing entrance, etc. Since the sweetness of the erythritol is 60-70% of the sweetness of sucrose, compared with the sucrose, the sweetness of the erythritol is unspectacular, the taste of the erythritol is thin, and the erythritol has a delayed bitterness. Therefore, in order to cover up these defects when the erythritol is used alone, and satisfy people's requirements for good taste and low calorie, high-intensity sweeteners or other sugar alcohols are often used to be compounded or co-crystallized with the erythritol in the industry.

At present, processing manners, which include performing coating spray on the surface of the erythritol by using a spray granulation technique, directly using a mixed sugar solution of the erythritol and other high-intensity sweeteners for spray granulation, etc., have been adopted. These processing manners have problems, such as coating falling off, uneven taste, etc. Alternatively, a co-crystallization product is obtained by using a co-crystallization technique to perform a cooling crystallization or melt crystallization on the erythritol and other high-intensity sweeteners. For example, in the Chinese Patent Application No. CN110179096A, a compound sweetener was obtained by performing a liquid cooling crystallization on the erythritol, inulin, stevioside, and mogroside for compound crystallization. The particle size of the compound sweetener is consistent, and the taste of the compound sweetener is good. In the Chinese Patent Application No. CN103262972A, co-crystallization crystals were obtained by performing the melt crystallization on the erythritol and sucralose for co-crystallization. The co-crystallization crystals have a good instant solubility and a uniform sweetness. US 2013/344216 A1 and CN 113 248 551 A are further relevant prior art documents. However, none of the manners whether using the cooling crystallization or the melt crystallization, disclose whether the particle sizes of products of the compound crystals can be regulated, which is not conducive to the preparation of the products with different particle size specifications. If the particle size specification of the products is required, the products prepared by the existing manners can only be sieved through a sieving treatment to obtain the products with the required particle size, which increases a preparation cost.

### SUMMARY

The invention relates to a system and a method for preparing a compound crystal of erythritol and a high-intensity sweeteners according to appended claims 1 and 3 respectively. The compound crystal of the erythritol and the high-intensity sweetener is obtained by using an evaporation crystallization. By using a regulatory manner that coordinates an added particle size of erythritol crystal seeds and a stirring speed, the particle size of the obtained compound crystals is concentrated and controllable, which is conducive to the preparation of products with different particle size specifications, thereby reducing the preparation cost. At the same time, the flavor of the erythritol can be improved, and the taste of the erythritol can be uniform, which is more consistent with consumer preferences.

The present invention relates to a system for preparing the compound crystal of the erythritol and the high-intensity sweetener according to claim 1. The system comprises a fermentation liquid storage tank, a ceramic membrane filtration system, a nanofiltration system, a first evaporator, a first crystallization kettle, a first centrifuge, a sugar dissolution tank, a decolorization tank, an ion exchange column, a blending tank, a second evaporator, a second crystallization kettle, a second centrifuge, a hot-air drying tank, a fluidized drying bed, and a finished product tank sequentially connected through pipelines. The system further comprises a high-intensity sweetener storage tank, a primary mother liquor tank, and a secondary mother liquor tank. The finished product tank stores a prepared compound crystallization product of the erythritol and the high-intensity sweetener.

The present invention relates to a method for preparing the compound crystal of the erythritol and the high-intensity sweetener according to claim 3. The method uses the system for preparing the compound crystal of the erythritol and the high-intensity sweetener according to claim 1 or claim 2. The method includes the following operations.

Operation 1, a penetrating liquid is obtained by sequentially conveying the sterilized erythritol fermentation liquid stored in the fermentation liquid storage tank to the ceramic membrane filtration system for filtration and the nanofiltration system for separation, and a solid erythritol monocrystalline sugar and a liquid primary mother liquor may be obtained, respectively, by conveying the penetrating liquid to the first evaporator for concentration, the first crystallization kettle for crystallization, and the first centrifuge for centrifugation through the pipelines. The primary mother liquor is temporarily stored in the primary mother liquor tank and then returned to the first evaporator through the pipeline for reuse. In operation 1, a content of the erythritol in the penetrating liquid after the separation performed by the nanofiltration system is larger than 93%, and a light transmittance of the erythritol in the penetrating liquid may be larger than 85%.

Operation 2, a dissolved liquid is obtained by dissolving the erythritol monocrystalline sugar in the dissolving sugar tank, a decolorization operation through the decolorization tank and an impurity removal operation through the ion exchange column is performed on the dissolved liquid sequentially, a blending liquid is obtained by mixing the processed dissolved liquid with the high-intensity sweetener liquid conveyed by the high-intensity sweetener storage tank and a secondary mother liquor conveyed by the secondary mother liquor tank, and then a solid compound moist sugar of the erythritol and the high-intensity sweetener, and the liquid secondary mother liquor is obtained, respectively, by sequentially introducing the blending liquid into the second evaporator for concentration, the second crystallization kettle for crystallization, and the second centrifuge for centrifugal separation through the pipelines. The secondary mother liquor is temporarily stored in the secondary mother liquor tank and then returned to the blending tank through the pipeline for reuse. In operation 2, a light transmittance of the processed dissolved liquid is larger than 97% and a conductivity of the processed dissolved liquid is less than 30 microsecond per centimeter (µs/cm). A refractive index of a blending concentrate after the concentration is performed on the blending liquid by the second evaporator is within a range from 65 to 70%, an erythritol content of the blending concentrate may be within a range from 95 to 99.9%, and a high-intensity sweetener content of the blending concentrate is within a range from 0.1 to 5%.

Operation 3, the prepared compound crystallization product of the erythritol and the high-intensity sweetener temporarily stored in the finished product tank is obtained by sequentially performing a hot-air drying operation through the hot-air drying tank and a cold-air drying operation through the fluidized drying bed on the obtained compound moist sugar of the erythritol and the high-intensity sweetener through the pipelines.

The system and the method for preparing the compound crystal of the erythritol and the high-intensity sweetener of the present disclosure have the following advantages.
1. The particle size of the compound crystal of the erythritol and the high-intensity sweetener obtained according to the present disclosure is concentrated and controllable.
2. A shape of the compound crystal of the erythritol and the high-intensity sweetener obtained according to the present disclosure is similar to that of an erythritol crystal, and a crystal form of the compound crystal is relatively good.
3. The sweetness of the compound crystal of the erythritol and the high-intensity sweetener obtained according to the present disclosure may be adjustable, which is within a range of 0.7 to 2 times of the sweetness of sucrose, and the taste of the compound crystal is uniform and better than that of the erythritol crystal.

### BRIEF DESCRIPTION OF THE DRAWINGS

FIG. 1 is a schematic diagram illustrating a system for preparing a compound crystal erythritol and a high-intensity sweetener according to the present invention;
FIG. 2 is a schematic diagram illustrating a particle size distribution for preparing a compound crystallization product of erythritol and a high-intensity sweetener according to an embodiment of the present invention.

### DETAILED DESCRIPTION

In order to more clearly illustrate the technical problems, the technical solutions, and beneficial effects of the embodiments of the present disclosure, the accompanying drawings to be used in the description of the embodiments will be briefly described below. It should be understood that the specific embodiments described herein are merely for illustrating the present disclosure and are not intended to limit the present invention as defined in the appended claims.

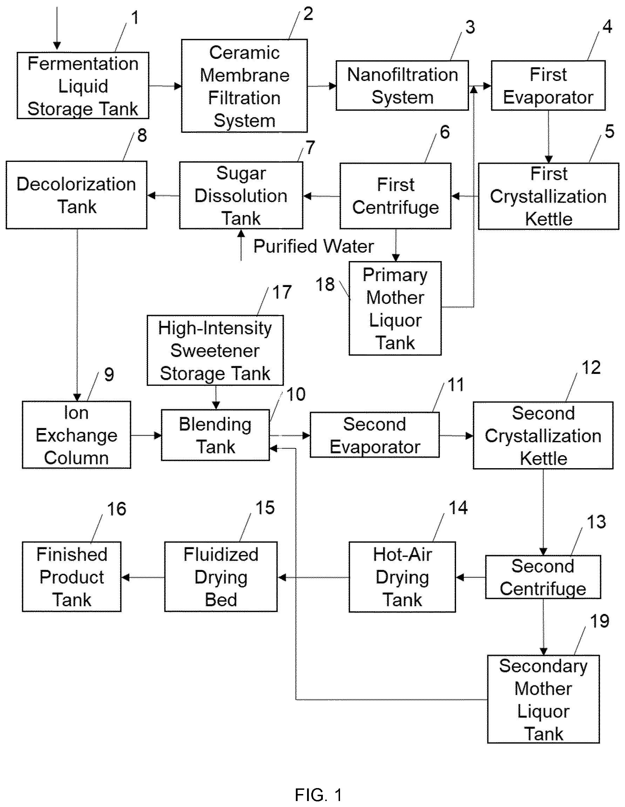

Referring to FIG. 1, the system for preparing the compound crystal of the erythritol and the high-intensity sweetener includes a fermentation liquid storage tank 1, a ceramic membrane filtration system 2, a nanofiltration system 3, a first evaporator 4, a first crystallization kettle 5, a first centrifuge 6, a sugar dissolution tank 7, a decolorization tank 8, an ion exchange column 9, a blending tank 10, a second evaporator 11, and a second crystallization kettle 12, a second centrifuge 13, a hot-air drying tank 14, a fluidized drying bed 15, and a finished product tank 16 sequentially connected through pipelines. The system further includes a high-intensity sweetener storage tank 17, a primary mother liquor tank 18, and a secondary mother liquor tank 19. Directions of arrows in the FIG. 1 indicate flow directions of materials in the system.

The fermentation liquid storage tank 1 is disposed with a feed inlet for a sterilized erythritol fermentation liquid, the nanofiltration system 3 is disposed with a penetrating liquid feed inlet, and the penetrating liquid feed inlet is connected to a feed inlet of the first evaporator 4 through a pipeline. The sugar dissolution tank 7 is disposed with a feed inlet for purified water, and the blending tank 10 is disposed with a feed inlet for a high-intensity sweetener.

Each of the first centrifuge 6 and the second centrifuge 13 is disposed with a solid material feed outlet and a liquid material feed outlet. The liquid material feed outlet of the first centrifuge 6 is connected to a feed inlet of the primary mother liquor tank 18 through the pipeline, and a feed outlet of the primary mother liquor tank 18 is connected to a feed inlet of the first evaporator 4 through a pipeline. The liquid material feed outlet of the second centrifuge 13 is connected to a feed inlet of the secondary mother liquor tank 19 through a pipeline, and a feed outlet of the secondary mother liquor tank 19 is connected to a feed inlet of the blending tank 10 through a pipeline. The finished product tank stores a prepared compound crystallization product of the erythritol and the high-intensity sweetener.

The sterilized erythritol fermentation liquid enters the fermentation liquid storage tank 1 through the feed inlet for temporary storage. The sterilized erythritol fermentation liquid is filtered by the ceramic membrane filtration system 2 and separated by the nanofiltration system 3, respectively, to remove most of impurities and obtain a penetrating liquid, and then an erythritol monocrystalline sugar and a primary mother liquor are obtained by sequentially conveying the penetrating liquid to the first evaporator 4 for evaporation and concentration, the first crystallization kettle 5 for crystallization, and the first centrifuge 6 for centrifugation through the pipelines. A dissolved liquid is obtained by conveying the erythritol monocrystalline sugar to the dissolving sugar tank 7 through the pipeline and dissolving the erythritol monocrystalline sugar in the dissolving sugar tank 7. A decolorization operation through the decolorization tank 8 and an impurity removal operation through the ion exchange column 9 are performed on the dissolved liquid sequentially. A blending liquid is obtained by mixing the processed dissolved liquid with the high-intensity sweetener liquid conveyed by the high-intensity sweetener storage tank 17 and a secondary mother liquor conveyed by the secondary mother liquor tank 19 in the blending tank 10, and then the compound crystallization product of the erythritol and the high-intensity sweetener is obtained by sequentially introducing the blending liquid into the second evaporator 11 for concentration, the second crystallization kettle 12 for crystallization, the second centrifuge 13 for centrifugal separation, the hot-air drying tank 14 for the hot-air drying, and the fluidized drying bed 15 for the cold-air drying.

The high-intensity sweetener may include at least one of stevioside, mogroside, sucralose, acesulfame, or aspartame.

The present invention also relates to a method for preparing a compound crystal of erythritol and a high-intensity sweetener. The method uses the system for preparing the compound crystal of the erythritol and the high-intensity sweetener according to claim 1 or claim 2. The method includes the following operations.

In operation 1, a penetrating liquid may be obtained by sequentially conveying the sterilized erythritol fermentation liquid stored in the fermentation liquid storage tank 1 to the ceramic membrane filtration system 2 for filtration and the nanofiltration system 3 for separation, and a solid erythritol monocrystalline sugar and a liquid primary mother liquor may be obtained, respectively, by conveying the penetrating liquid to the first evaporator 4 for concentration, the first crystallization kettle 5 for crystallization, and the first centrifuge 6 for centrifugation through the pipelines. The primary mother liquor is temporarily stored in the primary mother liquor tank 18 and then returned to the first evaporator 4 through the pipeline for reuse. In operation 1, a content of the erythritol in the penetrating liquid after the separation performed by the nanofiltration system 3 is larger than 93%, and a light transmittance of the erythritol in the penetrating liquid is larger than 85%.

In operation 2, a dissolved liquid is obtained by dissolving the erythritol monocrystalline sugar in the dissolving sugar tank 7. A decolorization operation through the decolorization tank 8 and an impurity removal operation through the ion exchange column 9 may be performed on the dissolved liquid sequentially. A blending liquid is obtained by mixing the processed dissolved liquid with the high-intensity sweetener liquid conveyed by the high-intensity sweetener storage tank 17 and a secondary mother liquor conveyed by the secondary mother liquor tank 19. Then a solid compound moist sugar of the erythritol and the high-intensity sweetener, and the liquid secondary mother liquor is obtained, respectively, by sequentially introducing the blending liquid into the second evaporator 11 for concentration, the second crystallization kettle 12 for crystallization, and the second centrifuge 13 for centrifugal separation through the pipelines. The secondary mother liquor is temporarily stored in the secondary mother liquor tank 19 and then returned to the blending tank 10 through the pipeline for reuse. In operation 2, a light transmittance of the processed dissolved liquid is larger than 97% and a conductivity of the processed dissolved liquid is less than 30µs/cm. A refractive index of a blending concentrate after the concentration is performed on the blending liquid by the second evaporator is within a range from 65 to 70%, an erythritol content of the blending concentrate is within a range from 95 to 99.9%, and a high-intensity sweetener content of the blending concentrate is within a range from 0.1 to 5%.

In operation 3, the prepared compound crystallization product of the erythritol and the high-intensity sweetener temporarily stored in the finished product tank 16 is obtained by sequentially performing a hot-air drying operation through the hot-air drying tank 14 and a cold-air drying operation through the fluidized drying bed 15 on the obtained compound moist sugar of the erythritol and the high-intensity sweetener through the pipelines.

In some embodiments, in operation 1, a refraction of the erythritol concentrate obtained after the concentration is performed on the penetrating liquid by the first evaporator 4 is within a range from 55 to 60%, and a crystallization temperature of the first crystallization kettle 5 is within a range from 60 to 65°C.

According to the invention, in operation 2, when the crystallization is performed in the second crystallization kettle 12, a stirring speed is set to be within a range from 50 to 100 revolutions per minute (rpm), and a crystallization temperature is set to be within a range from 60 to 65°C. After the erythritol crystal seeds are added, and the erythritol crystal seeds are dispersed uniformly in a sugar liquid, the crystallization temperature and the stirring speed are maintained constant, and the crystallization is cultivated for 0.5 to 2 hours (h). After the cultivation of the crystallization, a pressure of the second crystallization kettle 12 is set to be within a range from 150 to 220 millibar (mbar), and an evaporation crystallization is performed on the sugar liquid while stirring. After the evaporation crystallization is performed for 2 to 6 hours, the evaporation crystallization is stopped and crystals are out of the second crystallization kettle 12. The addition ratio of the erythritol crystal seeds is within a range from 1 to 5%, and the particle size of the erythritol crystal seeds is within a range from 0.250 mm to 0.125 mm (60 to 120 mesh).

In some embodiments, in operation 3, the hot-air temperature in the hot-air drying tank 14 is within a range from 90 to 100°C, and the cold-air temperature in the fluidized drying bed 15 is within a range from 15 to 30°C.

The system and the method for preparing the compound crystal of the erythritol and the high-intensity sweetener in the present disclosure are further described below according to specific embodiments.

### Embodiment 1

The embodiment 1 of the process for preparing the compound crystal of the erythritol and the high-intensity sweetener of the present disclosure may be performed a processor. The method includes the following operations.

(11) A sterilized erythritol fermentation liquid sequentially passed through the ceramic membrane filtration system 2 and the nanofiltration system 3, and a nanofiltrate with an erythritol content of 93% and a light transmittance of 85% was obtained by removing the bacteria and other impurities. The nanofiltrate was concentrated through the first evaporator 4 to obtain an erythritol concentrate with a refraction of 55%. The erythritol concentrate was evaporated and crystallized in the first crystallization kettle 5 to obtain an erythritol sugar paste. A crystallization temperature of the first crystallization kettle 5 was 60°C. The erythritol sugar paste was centrifuged through the first centrifuge 6 to obtain an erythritol monocrystalline sugar and a primary centrifugation mother liquor. The primary centrifugation mother liquor was returned for nanofiltration and then reused.

(12) The erythritol monocrystalline sugar was introduced into the dissolving sugar tank 7 for dissolution, and a dissolved liquid was obtained. The dissolved liquid sequentially passed through the decolorization tank 8 and the ion exchange column 9 to obtain a decolorization separation liquid with a light transmittance of 98% and a conductivity of 18 µs/cm. A blending liquid was obtained by mixing the decolorization separation liquid and a stevioside liquid in the blending tank 10 at a blending proportion. An erythritol content in the blending liquid is 99.84%, and a stevioside content in the blending liquid is 0.1%. The blending liquid was introduced into the second evaporator 11 for the concentration to obtain a blending concentrate of the erythritol and the stevioside with a refraction of 66%.

(13) The blending concentrate was introduced into the second crystallization kettle 12. A stirring speed was set to 60rpm, a temperature of the second crystallization kettle 12 was set to 65°C. Erythritol crystal seeds were added into the second crystallization kettle 12. An addition ratio of the erythritol crystal seeds was 2%, and a particle size of the erythritol crystal seeds was within a range from 0.125 mm to 0.177 mm (from 60 to 80 mesh). After the erythritol crystal seeds were dispersed uniformly in a sugar liquid, the temperature and stirring speed were maintained constant, and the crystallization was cultivated for 1 h. After the cultivation of the crystallization, a pressure of the second crystallization kettle 12 was set to 18 millibar (mbar), and an evaporation crystallization was performed on the sugar liquid while stirring. When the evaporation crystallization was performed for 4 h, the crystallization was stopped, and crystals were out of the second crystallization kettle 12 to obtain a compound crystallization sugar paste of the erythritol and the stevioside.

(14) The compound crystallization sugar paste of the erythritol and the stevioside was separated by the second centrifuge 13 to obtain compound crystals of the erythritol and the stevioside and a secondary mother liquor. Compound crystallization products of the erythritol and the stevioside were obtained by performing a hot-air drying operation at 90°C and a cold-air drying operation at 30°C on the compound crystals, and the secondary mother liquor (or the secondary centrifugal mother liquor) was returned to the blending tank 10 for reuse.

A particle size distribution of the compound crystallization products was obtained by sieving the compound crystallization products of the erythritol and the stevioside obtained in embodiment 1. The particle size distribution may be shown in Table 1.

**Table 1. Particle Size Distribution of Compound Crystallization Products of Erythritol and Stevioside of Embodiment 1, wherein:10-20 mesh = 2-0.841 mm; 20-30 mesh = 0.841-0.595 mm; 30-40 mesh = 0.595-0.4 mm; 40-50 mesh = 0-5-0.297 mm; 50-60 mm = 0.297-0.25 mm; less than 60 mesh = less than 0.25 mm.**

| Particle size | 10~20 mesh | 20~30 mesh | 30~40 mesh | 40~50 mesh | 50~60 mesh | Less than 60 mesh |
|---|---|---|---|---|---|---|
| Percentage/% | 42.68 | 28.01 | 17.43 | 8.38 | 1.49 | 2.01 |

As Table 1, the percentage of the compound crystallization products of the erythritol and the stevioside with the particle size from 2 mm to 0.595 mm (from 10 to 30 mesh) was 70.69%.

### Embodiment 2

The embodiment 2 of the process for preparing the compound crystal of the erythritol and the high-intensity sweetener of the present disclosure includes the following operations.

(21) A sterilized erythritol fermentation liquid sequentially passed through the ceramic membrane filtration system 2 and the nanofiltration system 3, and a nanofiltrate with an erythritol content of 96% and a light transmittance of 87% was obtained by removing the bacteria and other impurities. The nanofiltrate was concentrated through the first evaporator 4 to obtain an erythritol concentrate with a refraction of 60%. The erythritol concentrate was evaporated and crystallized in the first crystallization kettle 5 to obtain an erythritol sugar paste. A crystallization temperature of the first crystallization kettle 5 was 60°C. The erythritol sugar paste was centrifuged through the first centrifuge 6 to obtain an erythritol monocrystalline sugar and a primary centrifugation mother liquor. The primary centrifugation mother liquor was returned for nanofiltration and then reused.

(22) The erythritol monocrystalline sugar was introduced into the dissolving sugar tank 7 for dissolution, and a dissolved liquid was obtained. The dissolved liquid sequentially passed through the decolorization tank 8 and the ion exchange column 9 to obtain a decolorization separation liquid with a light transmittance of 99% and a conductivity of 25 µs/cm. A blending liquid was obtained by mixing the decolorization separation liquid and a stevioside liquid in the blending tank 10 at a blending proportion. An erythritol content in the blending liquid is 99.11%, a stevioside content in the blending liquid is 0.64%, and a mogroside content in the blending liquid is 0.25%. The blending liquid was introduced into the second evaporator 11 for the concentration to obtain a blending concentrate of the erythritol and the stevioside with a refraction of 70%.

(23) The blending concentrate was introduced into the second crystallization kettle 12. A stirring speed was set to 100rpm, a temperature of the second crystallization kettle 12 was set to 64°C. Erythritol crystal seeds were added into the second crystallization kettle 12. An addition ratio of the erythritol crystal seeds was 5%, and a particle size of the erythritol crystal seeds was within a range from 100 to 120 mesh. After the erythritol crystal seeds were dispersed uniformly in a sugar liquid, the temperature and stirring speed were maintained constant, and the crystallization was cultivated for 0.5 h. After the cultivation of the crystallization, a pressure of the second crystallization kettle 12 was set to 20 millibar (mbar), and an evaporation crystallization was performed on the sugar liquid while stirring. When the evaporation crystallization was performed for 6 h, the crystallization was stopped, and crystals were out of the second crystallization kettle 12 to obtain a compound crystallization sugar paste of the erythritol and the stevioside.

(24) The compound crystallization sugar paste of the erythritol and the stevioside was separated by the second centrifuge 13 to obtain compound crystals of the erythritol and the stevioside and a secondary mother liquor. Compound crystallization products of the erythritol and the stevioside were obtained by performing a hot-air drying operation at 95°C and a cold-air drying operation at 22°C on the compound crystals, and the secondary mother liquor (or the secondary centrifugal mother liquor) was returned to the blending tank 10 for reuse.

A particle size distribution of the compound crystallization products was obtained by sieving the compound crystallization products of the erythritol and the stevioside obtained in embodiment 2. The particle size distribution may be shown in Table 2.

**Table 2. Particle Size Distribution of Compound Crystallization Products of Erythritol and Stevioside of Embodiment 2, wherein mesh ranges correspond to ranges in mm as indicated above for Table 1.**

| Particle size | 10~20 mesh | 20~30 mesh | 30~40 mesh | 40~50 mesh | 50~60 mesh | Less than 60 mesh |
|---|---|---|---|---|---|---|
| Percentage/% | 1.5 | 3.68 | 18.62 | 38.83 | 23.24 | 14.12 |

As Table 2, the percentage of the compound crystallization products of the erythritol and the stevioside with the particle size less than 0.4 mm (less than 40 mesh) was 76.2%.

### Embodiment 3

The embodiment 3 of the process for preparing the compound crystal of the erythritol and the high-intensity sweetener of the present disclosure includes the following operations.

(31) A sterilized erythritol fermentation liquid sequentially passed through the ceramic membrane filtration system 2 and the nanofiltration system 3, and a nanofiltrate with an erythritol content of 95% and a light transmittance of 90% was obtained by removing the bacteria and other impurities. The nanofiltrate was concentrated through the first evaporator 4 to obtain an erythritol concentrate with a refraction of 58%. The erythritol concentrate was evaporated and crystallized in the first crystallization kettle 5 to obtain an erythritol sugar paste. A crystallization temperature of the first crystallization kettle 5 was 65°C. The erythritol sugar paste was centrifuged through the first centrifuge 6 to obtain an erythritol monocrystalline sugar and a primary centrifugation mother liquor. The primary centrifugation mother liquor was returned for nanofiltration and then reused.

(32) The erythritol monocrystalline sugar was introduced into the dissolving sugar tank 7 for dissolution, and a dissolved liquid was obtained. The dissolved liquid sequentially passed through the decolorization tank 8 and the ion exchange column 9 to obtain a decolorization separation liquid with a light transmittance of 99% and a conductivity of 20 µs/cm. A blending liquid was obtained by mixing the decolorization separation liquid and a stevioside liquid in the blending tank 10 at a blending proportion. An erythritol content in the blending liquid is 95.00%, a sucralose content in the blending liquid is 0.88%, a stevioside content in the blending liquid is 1.12%, and a mogroside content in the blending liquid is 3.0%. The blending liquid was introduced into the second evaporator 11 for the concentration to obtain a blending concentrate of the erythritol and the stevioside with a refraction of 68%.

(33) The blending concentrate was introduced into the second crystallization kettle 12. A stirring speed was set to 85rpm, a temperature of the second crystallization kettle 12 was set to 65°C. Erythritol crystal seeds were added into the second crystallization kettle 12. An addition ratio of the erythritol crystal seeds was 1%, and a particle size of the erythritol crystal seeds was within a range from 80 to 100 mesh. After the erythritol crystal seeds were dispersed uniformly in a sugar liquid, the temperature and stirring speed were maintained constant, and the crystallization was cultivated for 2 h. After the cultivation of the crystallization, a pressure of the second crystallization kettle 12 was set to 15 millibar (mbar), and an evaporation crystallization was performed on the sugar liquid while stirring. When the evaporation crystallization was performed for 2 h, the crystallization was stopped, and crystals were out of the second crystallization kettle 12 to obtain a compound crystallization sugar paste of the erythritol and the stevioside.

(34) The compound crystallization sugar paste of the erythritol and the stevioside was separated by the second centrifuge 13 to obtain compound crystals of the erythritol and the stevioside and a secondary mother liquor. Compound crystallization products of the erythritol and the stevioside were obtained by performing a hot-air drying operation at 100°C and a cold-air drying operation at 15°C on the compound crystals, and the secondary mother liquor (or the secondary centrifugal mother liquor) was returned to the blending tank 10 for reuse.

A particle size distribution of the compound crystallization products was obtained by sieving the compound crystallization products of the erythritol and the stevioside obtained in embodiment 3. The particle size distribution may be shown in Table 3.

**Table 3. Particle Size Distribution of Compound Crystallization Products of Erythritol and Stevioside of Embodiment 3, wherein mesh ranges correspond to ranges in mm as indicated above for Table 1.**

| Particle size | Larger than 20 | 20~30 mesh | 30~40 mesh | 40~50 mesh | 50~60 mesh | Less than 60 mesh |
|---|---|---|---|---|---|---|
| Percentage/% | 2.42 | 5.76 | 27.92 | 40.01 | 8.49 | 15.40 |

As Table 3 the percentage of the compound crystallization products of the erythritol and the stevioside with the particle size from 0.595 to 0.297 (from 30 to 50 mesh) was 67.93%.

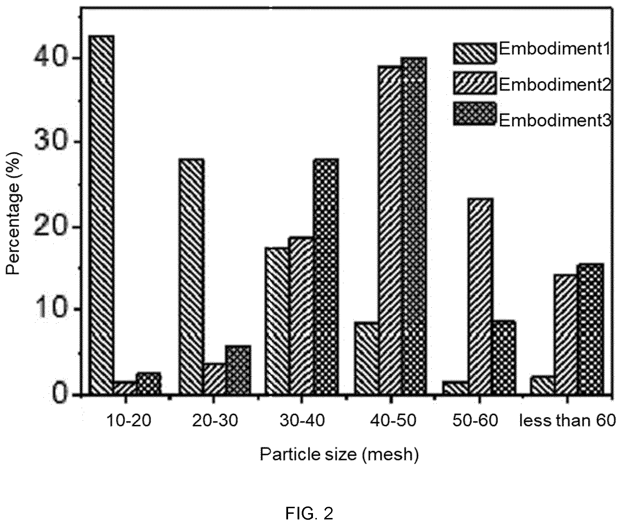

A particle size distribution diagram shown in FIG. 2 can be obtained by collating the particle size distributions of the compound crystallization products of the erythritol and the stevioside prepared in embodiments 1 to 3.

A taste test was performed on the compound crystallized products of the erythritol and the stevioside obtained according to embodiments 1-3, respectively. Taste comparison tests were also performed on the compound crystallized products of the erythritol and the stevioside obtained according to embodiments 1-3 with sucrose products and stevioside products. Each of the sucrose products and the stevioside products was configured to a sugar liquid with a concentration of 7%. An intensity of a sweetness of a crystal in which a concentration of the sucrose is 7% was defaulted to 7. In some embodiments, the intensity of each taste can be indicated by a numerical value. The larger the numerical value, the larger the intensity of the type of taste. For example, the intensity of the taste can include 7 grades, wherein a value "1" represents very weak, a value "2" represents relatively weak, a value "3" represents -somewhat weak, a value "4" represents average, a value "5" represents somewhat strong, a value "6" represents relatively strong, and a value "7" represents very strong. Test results were obtained as shown in Table 4.

**Table 4. Taste Test Results For Products**

| Items | Sucrose | Stevioside | Embodiment 1 | Embodiment 2 | Embodiment 3 |
|---|---|---|---|---|---|
| Sweetness | 7 | 4.9 | 6.2 | 7.3 | 8.9 |
| Bitterness | 1.7 | 2.3 | 2.1 | 1.8 | 1.9 |
| Metallic Taste | 1 | 1.4 | 1.5 | 1.2 | 1.4 |
| Delayed Sweetness | 2.5 | 1.8 | 2.7 | 2.8 | 3.1 |
| Delayed Bitterness | 1.7 | 1.9 | 1.6 | 1.6 | 1.8 |
| Acerbic Taste | 1.9 | 2.1 | 2.0 | 1.9 | 2.3 |

## Claims

1. A system for preparing a compound crystal of erythritol and a high-intensity sweetener, wherein the system is configured to perform evaporation crystallization and to coordinate an added particle size of erythritol crystal seeds and a stirring speed, wherein the system comprises a fermentation liquid storage tank (1), a ceramic membrane filtration system (2), a nanofiltration system (3), a first evaporator (4), a first crystallization kettle (5), a first centrifuge (6), a sugar dissolution tank (7), a decolorization tank (8), an ion exchange column (9), a blending tank (10), a second evaporator (11), a second crystallization kettle (12), a second centrifuge (13), a hot-air drying tank (14), a fluidized drying bed (15), and a finished product tank (16) sequentially connected through pipelines, and
the system further comprises a high-intensity sweetener storage tank (17), a primary mother liquor tank (18), and a secondary mother liquor tank (19), wherein
the fermentation liquid storage tank (1) is disposed with a feed inlet for sterilized erythritol fermentation liquid,
the nanofiltration system (3) is disposed with a penetrating liquid feed outlet,
the penetrating liquid feed outlet is connected to a feed inlet of the first evaporator (4) through a pipeline,
the sugar dissolution tank (7) is disposed with a feed inlet for purified water,
the blending tank (10) is disposed with a feed inlet for the high-intensity sweetener,
each of the first centrifuge (6) and the second centrifuge (13) is disposed with a solid material feed outlet and a liquid material feed outlet,
the liquid material feed outlet of the first centrifuge (6) is connected to a feed inlet of the primary mother liquor tank (18) through a pipeline,
a feed outlet of the primary mother liquor tank (18) is connected to the feed inlet of the first evaporator (4) through a pipeline,
the liquid material feed outlet of the second centrifuge (13) is connected to a feed inlet of the secondary mother liquor tank (19) through a pipeline,
a feed outlet of the secondary mother liquor tank (19) is connected to a feed inlet of the blending tank (10) through a pipeline, and
the finished product tank (16) stores a prepared compound crystallization product of the erythritol and the high-intensity sweetener.

2. The system of claim 1, wherein the high-intensity sweetener includes at least one of stevioside, mogroside, sucralose, acesulfame, or aspartame.

3. A method for preparing a compound crystal of erythritol and a high-intensity sweetener, wherein the method uses the system according to claim 1 or claim 2, the method comprising:
operation 1, obtaining a penetrating liquid by sequentially conveying the sterilized erythritol fermentation liquid stored in the fermentation liquid storage tank (1) to the ceramic membrane filtration system (2) for filtration and the nanofiltration system (3) for separation, and obtaining a solid erythritol monocrystalline sugar and a liquid primary mother liquor, respectively, by conveying the penetrating liquid to the first evaporator (4) for concentration, the first crystallization kettle (5) for crystallization, and the first centrifuge (6) for centrifugation through the pipelines, wherein
the primary mother liquor is temporarily stored in the primary mother liquor tank (18) and then returned to the first evaporator (4) through the pipeline for reuse, and
in operation 1, a content of the erythritol in the penetrating liquid after the separation performed by the nanofiltration system (3) is larger than 93%, and a light transmittance of the erythritol in the penetrating liquid is larger than 85%;
operation 2, obtaining a dissolved liquid by dissolving the erythritol monocrystalline sugar in the dissolving sugar tank, performing a decolorization operation through the decolorization tank (8) and an impurity removal operation through the ion exchange column (9) on the dissolved liquid sequentially, obtaining a blending liquid by mixing the processed dissolved liquid with the high-intensity sweetener liquid conveyed by the high-intensity sweetener storage tank (17) and a secondary mother liquor conveyed by the secondary mother liquor tank (19), and then obtaining a solid compound moist sugar of the erythritol and the high-intensity sweetener, and the liquid secondary mother liquor, respectively, by sequentially introducing the blending liquid into the second evaporator (11) for concentration, the second crystallization kettle (12) for crystallization, and the second centrifuge (13) for centrifugal separation through the pipelines, wherein
the secondary mother liquor is temporarily stored in the secondary mother liquor tank (19) and then returned to the blending tank (10) through the pipeline for reuse,
in operation 2, a light transmittance of the processed dissolved liquid is larger than 97% and a conductivity of the processed dissolved liquid is less than 30 microsecond per centimeter (µs/cm),
a refractive index of a blending concentrate after the concentration is performed on the blending liquid by the second evaporator (11) is within a range from 65 to 70%, an erythritol content of the blending concentrate is within a range from 95 to 99.9%, and a high-intensity sweetener content of the blending concentrate is within a range from 0.1 to 5%;
wherein, when the crystallization is performed in the second crystallization kettle (12), a stirring speed is set to be within a range from 50 to 100 revolutions per minute (rpm), and a crystallization temperature is set to be within a range from 60 to 65°C;
after erythritol crystal seeds are added, and the erythritol crystal seeds are dispersed uniformly in a sugar liquid, the crystallization temperature and the stirring speed are maintained constant, and the crystallization is cultivated for 0.5 to 2 hours (h);
after the cultivation of the crystallization, a pressure of the second crystallization kettle (12) is set to be within a range from 150 to 220 millibar (mbar), and an evaporation crystallization is performed on the sugar liquid while stirring,
after the evaporation crystallization is performed for 2 to 6 hours, the evaporation crystallization is stopped and crystals are out of the second crystallization kettle (12), an addition ratio of the erythritol crystal seeds being within a range from 1 to 5%, and a particle size of the erythritol crystal seeds being within a range from 0.250 mm to 0.125 mm (60 to 120 mesh), and
operation 3, obtaining the prepared compound crystallization product of the erythritol and the high-intensity sweetener temporarily stored in the finished product tank (16) by sequentially performing a hot-air drying operation through the hot-air drying tank (14) and a cold-air drying operation through the fluidized drying bed (15) on the obtained compound moist sugar of the erythritol and the high-intensity sweetener through the pipelines.

4. The method of claim 3, wherein in operation 1, a refractive index of an erythritol concentrate obtained after the concentration is performed on the penetrating liquid by the first evaporator (4) is within a range from 55 to 60%, and a crystallization temperature of the first crystallization kettle (5) is within a range from 60 to 65°C.

5. The method of claim 3, wherein in operation 3, a hot-air temperature in the hot-air drying tank (14) is within a range from 90 to 100°C, and a cold-air temperature in the fluidized drying bed (15) is within a range from 15 to 30°C.

## Patentansprüche

1. System zur Herstellung eines Verbundkristalls aus Erythrit und einem Hochintensitätssüßstoff, wobei das System dazu ausgebildet ist, eine Verdampfungskristallisation durchzuführen und eine zugesetzte Partikelgröße von Erythrit-Saatkristallen sowie eine Rührgeschwindigkeit aufeinander abzustimmen, wobei das System einen Fermentationsflüssigkeits-Speichertank (1), ein Keramikmembran-Filtrationssystem (2), ein Nanofiltrationssystem (3), einen ersten Verdampfer (4), einen ersten Kristallisationskessel (5), eine erste Zentrifuge (6), einen Zuckerauflösetank (7), einen Entfärbungstank (8), eine Ionenaustauschkolonne (9), einen Mischbehälter (10), einen zweiten Verdampfer (11), einen zweiten Kristallisationskessel (12), eine zweite Zentrifuge (13), einen Heißlufttrocknungstank (14), ein Wirbelschichttrocknungsbett (15) und einen Fertigprodukttank (16) umfasst, die der Reihe nach über Rohrleitungen miteinander verbunden sind, und
das System ferner einen Hochintensitätssüßstoff-Speichertank (17), einen Primärmutterlaugetank (18) und einen Sekundärmutterlaugetank (19) umfasst, wobei
der Fermentationsflüssigkeits-Speichertank (1) mit einem Zuführeinlass für sterilisierte Erythrit-Fermentationsflüssigkeit versehen ist,
das Nanofiltrationssystem (3) mit einem Permeatauslass versehen ist,
der Permeatauslass über eine Rohrleitung mit einem Zuführeinlass des ersten Verdampfers (4) verbunden ist,
der Zuckerauflösetank (7) mit einem Zuführeinlass für gereinigtes Wasser versehen ist,
der Mischbehälter (10) mit einem Zuführeinlass für den Hochintensitätssüßstoff versehen ist,
jeweils die erste Zentrifuge (6) und die zweite Zentrifuge (13) mit einem Feststoffauslass und einem Flüssigkeitsauslass versehen ist,
der Flüssigkeitsauslass der ersten Zentrifuge (6) über eine Rohrleitung mit einem Zuführeinlass des Primärmutterlaugetanks (18) verbunden ist,
ein Auslass des Primärmutterlaugetanks (18) über eine Rohrleitung mit dem Zuführeinlass des ersten Verdampfers (4) verbunden ist,
der Flüssigkeitsauslass der zweiten Zentrifuge (13) über eine Rohrleitung mit einem Zuführeinlass des Sekundärmutterlaugetanks (19) verbunden ist,
ein Auslass des Sekundärmutterlaugetanks (19) über eine Rohrleitung mit dem Zuführeinlass des Mischbehälters (10) verbunden ist, und
der Fertigprodukttank (16) ein hergestelltes Verbundkristallisationsprodukt aus dem Erythrit und dem Hochintensitätssüßstoff speichert.

2. System nach Anspruch 1, wobei der Hochintensitätssüßstoff mindestens eines aus der Gruppe bestehend aus Steviosid, Mogrosid, Sucralose, Acesulfam oder Aspartam umfasst.

3. Verfahren zur Herstellung eines Verbundkristalls aus Erythrit und einem Hochintensitätssüßstoff, wobei das Verfahren das System nach Anspruch 1 oder Anspruch 2 verwendet, wobei das Verfahren umfasst:
Vorgang 1, Erhalten eines Permeats, indem die in dem Fermentationsflüssigkeits-Speichertank (1) gespeicherte sterilisierte Erythrit-Fermentationsflüssigkeit der Reihe nach dem Keramikmembran-Filtrationssystem (2) zum Filtrieren und dem Nanofiltrationssystem (3) zum Trennen zugeführt wird, und Erhalten eines festen Erythrit-Monokristallzuckers sowie einer flüssigen Primärmutterlauge, jeweils, indem das Permeat über die Rohrleitungen dem ersten Verdampfer (4) zum Konzentrieren, dem ersten Kristallisationskessel (5) zum Kristallisieren und der ersten Zentrifuge (6) zum Zentrifugieren zugeführt wird, wobei
die Primärmutterlauge vorübergehend in dem Primärmutterlaugetank (18) zwischengespeichert und anschließend über die Rohrleitung zum ersten Verdampfer (4) zur Wiederverwendung zurückgeführt wird, und
in Vorgang 1 ein Gehalt an Erythrit im Permeat nach der durch das Nanofiltrationssystem (3) durchgeführten Trennung größer als 93% ist und eine Lichttransmission des Erythrits im Permeat größer als 85% ist;
Vorgang 2, Erhalten einer Lösung, indem der Erythrit-Monokristallzucker in dem Zuckerauflösetank gelöst wird, an der Lösung nacheinander ein Entfärbungsvorgang mittels des Entfärbungstanks (8) und ein Verunreinigungsentfernungsvorgang mittels der Ionenaustauschkolonne (9) durchgeführt wird, Erhalten einer Mischflüssigkeit durch Mischen der verarbeiteten Lösung mit einer durch den Hochintensitätssüßstoff-Speichertank (17) geförderten Hochintensitätssüßstoffflüssigkeit und einer durch den Sekundärmutterlaugetank (19) geförderten Sekundärmutterlauge, und anschließend Erhalten eines festen Verbund-Feuchtzuckers aus dem Erythrit und dem Hochintensitätssüßstoff sowie der flüssigen Sekundärmutterlauge, jeweils, indem die Mischflüssigkeit über die Rohrleitungen der Reihe nach in den zweiten Verdampfer (11) zum Konzentrieren, den zweiten Kristallisationskessel (12) zum Kristallisieren und die zweite Zentrifuge (13) zur Zentrifugaltrennung eingeleitet wird, wobei
die Sekundärmutterlauge vorübergehend in dem Sekundärmutterlaugetank (19) zwischengespeichert und anschließend über die Rohrleitung zum Mischbehälter (10) zur Wiederverwendung zurückgeführt wird,
in Vorgang 2 eine Lichttransmission der verarbeiteten Lösung größer als 97% ist und eine Leitfähigkeit der verarbeiteten Lösung kleiner als 30 Mikrosekunden pro Zentimeter (µs/cm) ist,
ein Brechungsindex eines Mischkonzentrats nach der durch den zweiten Verdampfer (11) an der Mischflüssigkeit durchgeführten Konzentration im Bereich von 65 bis 70% liegt, ein Erythritgehalt des Mischkonzentrats im Bereich von 95 bis 99.9% liegt, und ein Hochintensitätssüßstoffgehalt des Mischkonzentrats im Bereich von 0.1 bis 5% liegt;
wobei, wenn die Kristallisation in dem zweiten Kristallisationskessel (12) durchgeführt wird, eine Rührgeschwindigkeit im Bereich von 50 bis 100 Umdrehungen pro Minute (rpm) eingestellt wird und eine Kristallisationstemperatur im Bereich von 60 bis 65°C eingestellt wird;
nachdem Erythrit-Saatkristalle zugesetzt worden sind und die Erythrit-Saatkristalle in einer Zuckerlösung gleichmäßig dispergiert worden sind, die Kristallisationstemperatur und die Rührgeschwindigkeit konstant gehalten werden und die Kristallisation für 0.5 bis 2 Stunden (h) kultiviert wird;
nach der Kultivierung der Kristallisation ein Druck des zweiten Kristallisationskessels (12) im Bereich von 150 bis 220 Millibar (mbar) eingestellt wird und eine Verdampfungskristallisation der Zuckerlösung unter Rühren durchgeführt wird;
nachdem die Verdampfungskristallisation für 2 bis 6 Stunden durchgeführt worden ist, die Verdampfungskristallisation gestoppt wird und Kristalle aus dem zweiten Kristallisationskessel (12) ausgetragen werden, wobei ein Zugabeverhältnis der Erythrit-Saatkristalle im Bereich von 1 bis 5% liegt und eine Partikelgröße der Erythrit-Saatkristalle im Bereich von 0.250 mm bis 0.125 mm (60 bis 120 mesh) liegt; und
Vorgang 3, Erhalten des hergestellten Verbundkristallisationsprodukts aus dem Erythrit und dem Hochintensitätssüßstoff, das vorübergehend in dem Fertigprodukttank (16) zwischengespeichert ist, indem an dem erhaltenen Verbund-Feuchtzucker aus dem Erythrit und dem Hochintensitätssüßstoff über die Rohrleitungen der Reihe nach ein Heißlufttrocknungsvorgang mittels des Heißlufttrocknungstanks (14) und ein Kaltlufttrocknungsvorgang mittels des Wirbelschichttrocknungsbetts (15) durchgeführt werden.

4. Verfahren nach Anspruch 3, wobei in Vorgang 1 ein Brechungsindex eines Erythrit-Konzentrats, das nach der durch den ersten Verdampfer (4) an dem Permeat durchgeführten Konzentration erhalten wird, im Bereich von 55 bis 60% liegt, und eine Kristallisationstemperatur des ersten Kristallisationskessels (5) im Bereich von 60 bis 65°C liegt.

5. Verfahren nach Anspruch 3, wobei in Vorgang 3 eine Heißlufttemperatur in dem Heißlufttrocknungstank (14) im Bereich von 90 bis 100°C liegt und eine Kaltlufttemperatur in dem Wirbelschichttrocknungsbett (15) im Bereich von 15 bis 30°C liegt.

## Revendications

1. Système de préparation d'un cristal composé d'érythritol et d'un édulcorant à haute intensité, le système étant configuré pour effectuer une cristallisation par évaporation et pour coordonner une taille de particules des germes cristallins d'érythritol ajoutés et une vitesse d'agitation, le système comprenant un réservoir de stockage de liquide de fermentation (1), un système de filtration à membrane céramique (2), un système de nanofiltration (3), un premier évaporateur (4), une première cuve de cristallisation (5), une première centrifugeuse (6), une cuve de dissolution du sucre (7), une cuve de décoloration (8), une colonne d'échange ionique (9), une cuve de mélange (10), un second évaporateur (11), une seconde cuve de cristallisation (12), une seconde centrifugeuse (13), une cuve de séchage à air chaud (14), un lit de séchage fluidisé (15) et une cuve de produit fini (16) connectés séquentiellement par des conduites, et
le système comprenant en outre un réservoir de stockage d'édulcorant à haute intensité (17), une cuve de liqueur mère primaire (18) et une cuve de liqueur mère secondaire (19), dans lequel
le réservoir de stockage de liquide de fermentation (1) est muni d'une entrée d'alimentation pour un liquide de fermentation d'érythritol stérilisé,
le système de nanofiltration (3) est muni d'une sortie de perméat,
ladite sortie de perméat est reliée à une entrée d'alimentation du premier évaporateur (4) par une conduite,
la cuve de dissolution du sucre (7) est munie d'une entrée d'alimentation pour de l'eau purifiée,
la cuve de mélange (10) est munie d'une entrée d'alimentation pour l'édulcorant à haute intensité,
chacune de la première centrifugeuse (6) et de la seconde centrifugeuse (13) est munie d'une sortie de matière solide et d'une sortie de matière liquide,
la sortie de matière liquide de la première centrifugeuse (6) est reliée à une entrée d'alimentation de la cuve de liqueur mère primaire (18) par une conduite,
une sortie de la cuve de liqueur mère primaire (18) est reliée à l'entrée d'alimentation du premier évaporateur (4) par une conduite,
la sortie de matière liquide de la seconde centrifugeuse (13) est reliée à une entrée d'alimentation de la cuve de liqueur mère secondaire (19) par une conduite,
une sortie de la cuve de liqueur mère secondaire (19) est reliée à l'entrée d'alimentation de la cuve de mélange (10) par une conduite, et
la cuve de produit fini (16) stocke un produit de cristallisation composé préparé de l'érythritol et de l'édulcorant à haute intensité.

2. Système selon la revendication 1, dans lequel l'édulcorant à haute intensité comprend au moins un parmi le stévioside, le mogroside, le sucralose, l'acésulfame ou l'aspartame.

3. Procédé de préparation d'un cristal composé d'érythritol et d'un édulcorant à haute intensité, le procédé utilisant le système selon la revendication 1 ou la revendication 2, le procédé comprenant :
opération 1, obtention d'un perméat en convoyant successivement le liquide de fermentation d'érythritol stérilisé stocké dans le réservoir de stockage de liquide de fermentation (1) vers le système de filtration à membrane céramique (2) pour filtration puis vers le système de nanofiltration (3) pour séparation, et obtention, respectivement, d'un sucre monocristallin solide d'érythritol et d'une liqueur mère primaire liquide, en convoyant le perméat vers le premier évaporateur (4) pour concentration, la première cuve de cristallisation (5) pour cristallisation et la première centrifugeuse (6) pour centrifugation par les conduites, dans laquelle
la liqueur mère primaire est stockée temporairement dans la cuve de liqueur mère primaire (18) puis renvoyée vers le premier évaporateur (4) par la conduite pour réutilisation, et
dans l'opération 1, une teneur en érythritol dans le perméat après la séparation réalisée par le système de nanofiltration (3) est supérieure à 93% et une transmittance lumineuse de l'érythritol dans le perméat est supérieure à 85% ;
opération 2, obtention d'une solution en dissolvant le sucre monocristallin d'érythritol dans la cuve de dissolution du sucre, en réalisant successivement une opération de décoloration au moyen de la cuve de décoloration (8) et une opération d'élimination d'impuretés au moyen de la colonne d'échange ionique (9) sur la solution, obtention d'un liquide de mélange en mélangeant la solution traitée avec un liquide d'édulcorant à haute intensité convoyé par le réservoir de stockage d'édulcorant à haute intensité (17) et une liqueur mère secondaire convoyée par la cuve de liqueur mère secondaire (19), puis obtention, respectivement, d'un sucre humide composé solide d'érythritol et d'édulcorant à haute intensité et de la liqueur mère secondaire liquide, en introduisant successivement le liquide de mélange dans le second évaporateur (11) pour concentration, la seconde cuve de cristallisation (12) pour cristallisation et la seconde centrifugeuse (13) pour séparation centrifuge par les conduites, dans laquelle
la liqueur mère secondaire est stockée temporairement dans la cuve de liqueur mère secondaire (19) puis renvoyée vers la cuve de mélange (10) par la conduite pour réutilisation,
dans l'opération 2, une transmittance lumineuse de la solution traitée est supérieure à 97% et une conductivité de la solution traitée est inférieure à 30 microseconde par centimètre (µs/cm),
un indice de réfraction d'un concentré de mélange après la concentration réalisée sur le liquide de mélange par le second évaporateur (11) est dans une plage de 65 à 70%, une teneur en érythritol du concentré de mélange est dans une plage de 95 à 99.9% et une teneur en édulcorant à haute intensité du concentré de mélange est dans une plage de 0.1 à 5% ;
dans laquelle, lorsque la cristallisation est réalisée dans la seconde cuve de cristallisation (12), une vitesse d'agitation est réglée dans une plage de 50 à 100 tours par minute (rpm) et une température de cristallisation est réglée dans une plage de 60 à 65°C ;
après ajout de germes cristallins d'érythritol, et après dispersion uniforme desdits germes cristallins d'érythritol dans un liquide sucré, la température de cristallisation et la vitesse d'agitation sont maintenues constantes et la cristallisation est cultivée pendant 0.5 à 2 heures (h) ;
après la culture de la cristallisation, une pression de la seconde cuve de cristallisation (12) est réglée dans une plage de 150 à 220 millibar (mbar), et une cristallisation par évaporation est réalisée sur le liquide sucré tout en agitant ;
après réalisation de la cristallisation par évaporation pendant 2 à 6 heures, la cristallisation par évaporation est arrêtée et des cristaux sont sortis de la seconde cuve de cristallisation (12), un rapport d'ajout des germes cristallins d'érythritol étant dans une plage de 1 à 5% et une taille de particules des germes cristallins d'érythritol étant dans une plage de 0.250 mm à 0.125 mm (60 à 120 mesh) ; et
opération 3, obtention du produit de cristallisation composé préparé de l'érythritol et de l'édulcorant à haute intensité stocké temporairement dans la cuve de produit fini (16), en réalisant successivement une opération de séchage à air chaud au moyen de la cuve de séchage à air chaud (14) puis une opération de séchage à air froid au moyen du lit de séchage fluidisé (15) sur le sucre humide composé obtenu d'érythritol et d'édulcorant à haute intensité par les conduites.

4. Procédé selon la revendication 3, dans lequel, dans l'opération 1, un indice de réfraction d'un concentré d'érythritol obtenu après la concentration réalisée sur le perméat par le premier évaporateur (4) est dans une plage de 55 à 60% et une température de cristallisation de la première cuve de cristallisation (5) est dans une plage de 60 à 65°C.

5. Procédé selon la revendication 3, dans lequel, dans l'opération 3, une température d'air chaud dans la cuve de séchage à air chaud (14) est dans une plage de 90 à 100°C et une température d'air froid dans le lit de séchage fluidisé (15) est dans une plage de 15 à 30°C.
